# EUROPEAN PATENT APPLICATION

(11) **EP 4 353 810 A1**
(43) Date of publication of application: **17.04.2024**
(21) Application number: 21944442.9
(22) Date of filing: 07.06.2021
(51) Int. Cl.: C12M 1/00, C12Q 1/6844, C12Q 1/6848, C12Q 1/686

(54) **DNA DETECTION METHOD AND DNA DETECTION SYSTEM**

(71) Applicant: HITACHI HIGH-TECH CORPORATION, Tokyo 105-6409 (JP)
(72) Inventor: TANAKA, Junko, Tokyo 100-8280 (JP); NAKAGAWA, Tatsuo, Tokyo 100-8280 (JP); ISHIDA, Takeshi, Tokyo 100-8280 (JP); KAMURA, Yoshio, Tokyo 100-8280 (JP)
(74) Representative: Mewburn Ellis LLP
(86) International application number: PCT/JP2021/021633
(87) International publication number: WO 2022/259334

(57) **Abstract**

The present invention provides a DNA detection method comprising: a first step of dividing a specimen solution containing a fluorescent-labeled probe or a DNA intercalator and multiple types of DNA to be detected into a plurality of aliquots; a second step of performing PCR in microcompartments, each containing one of the aliquots; a third step of measuring fluorescence intensity from the fluorescent-labeled probe or the DNA intercalator in each of the microcompartments as temperature changes; a fourth step of calculating a melting temperature of the multiple type of DNA to be detected from each measured fluorescence intensity; a fifth step of identifying any microcompartment affected by one or more bubbles; and a sixth step of excluding data obtained for the microcompartment affected by one or more bubbles from all data obtained for the plurality of microcompartments.

## Description

### FIELD OF THE INVENTION

The present invention relates to a DNA detection method and a DNA detection system, and more particularly, to digital PCR.

### DESCRIPTION OF THE RELATED ART

There are known techniques for genetic testing such as PCR (see Patent Documents 2-4), and real-time PCR (See Non-Patent Document 1). In these methods, measurement reproducibility decreases when the amount of the gene to be detected (referred to as "target gene" herein) is small.

Digital PCR (see Patent Document 1) was developed as a technique for solving this problem. Digital PCR can be employed to quantify trace amounts of DNA by determining whether DNA is 0 (absent) or 1 (present) in samples that have been subjected to limiting dilution.

An exemplary method for quantifying DNA in a specimen using digital PCR is described below. First, when a PCR reaction solution is divided into wells of a plate or as droplets in oil, the sample is diluted so that each aliquot either contains one molecule of the target gene or none. The PCR reaction solution is then prepared by adding the DNA polymerase, primers and fluorescent-labelled probes required for PCR to the diluted samples. The resulting PCR reaction solution is divided into aliquots as described above.

Next, the target gene in each aliquot is amplified by PCR. After completion of PCR, the fluorescence intensity of each aliquot is measured, and the number of microcompartments with fluorescence intensity above a threshold is counted. The amount of the target gene in the specimen can be calculated from the obtained count.

In such digital PCR, the use of a limiting dilution of the specimen can reduce the influence of specimen-derived components that can inhibit PCR. In addition, the absolute amount of target DNA can be measured directly without a need for a calibration curve.

Incidentally, in conventional PCR, reaction efficiency is reduced due to the presence of a reaction inhibitor in the reaction solution, the formation of the secondary structures in the template DNA and inadequate primer design.

On the other hand, in the digital PCR, since DNA is detected as 0 or 1 at the endpoint of the reaction, the efficiency of PCR reaction itself has been considered to have no significant effect on the measurement result. In fact, however, even when measured at the endpoint, fluorescence intensity varies widely due to ununiform reaction efficiency among each aliquot. This was responsible for the poor measurement reproducibility and accuracy of digital PCR.

To improve the measurement reproducibility and accuracy of digital PCR, the inventors developed a technique to determine the presence or absence of the target gene in each aliquot, even if the PCR reaction efficiency among the aliquots is not uniform, by measuring the melting temperature (hereinafter, referred to as Tm) of a PCR amplicon through melting curve analysis(See Patent Document 5).

### PRIOR-ARTS

### PATENT DOCUMENTS

Patent Document 1: JP2013-521764
Patent Document 2: U.S. Pat. No. 4,683,195
Patent Document 3: U.S. Pat. No. 4,683,202
Patent Document 4: U.S. Pat. No. 4,800,159
Patent Document 5: JP2018-108063

### NON-PATENT DOCUMENTS

Non-Patent Document 1: Genome Res., 10, pp986-994, 1996

### SUMMARY OF THE INVENTION

### TECHNICAL PROBLEM

However, in digital PCR using melting curve analysis, if bubbles occur near the microcompartment containing the aliquots of the sample during fluorescence image acquisition for Tm calculation, the Tm value will be calculated incorrectly. In the digital PCR, the specimen is divided into aliquots and the target gene in the aliquots is amplified in the microcompartments arrayed in a plane. The fluorescence images of the microcompartments are acquired as temperature changes, and the Tm of the gene in the aliquot is calculated from the changes in the fluorescence intensity for each microcompartment. In this process, the bubbles are likely to be generated when the temperature is high during fluorescence image acquisition. In particular, the bubbles may generate in oil during PCR when PCR reaction solution is added to a through-hole that has been made as the microcompartment in a cartridge and sealed by oil or when droplets in oil are arranged in a plane in micro-channel. As the generated bubbles move during fluorescence image acquisition, the fluorescence intensity of each microcompartment fluctuates and causes noise in the melting curve. As a result, the calculated temperature differ from the melting temperature of the gene in the microcompartment, and the quantification of the target gene was not performed correctly in some cases.

The purpose of this invention is to provide a novel DNA detection system and DNA detection method and using melting curve analysis, by which the microcompartments located in the vicinity of generated bubbles with a measuring device are detected and target genes are accurately counted in digital PCR.

### MEANS TO SOLVE THE PROBLEM

In digital PCR using melting curve analysis, the inventors observed that the microcompartments with calculated melting temperatures that different from those of the genotype in the added specimen were unevenly distributed. They also identified the presence of the bubbles as the cause of deviation of melting temperatures and found that the erroneous judgment of the genotype can be reduced by excluding the microcompartments positioned near bubbles from analytical data.

In some embodiment, the present invention provides a DNA detection method comprising: a first step of dividing a specimen solution containing a fluorescent-labeled probe or a DNA intercalator and multiple types of DNA to be detected into a plurality of aliquots; a second step of performing PCR in microcompartments, each containing one of the aliquots; a third step of measuring fluorescence intensity from the fluorescent-labeled probe or the DNA intercalator in each of the microcompartments as temperature changes; a fourth step of calculating a melting temperature of the multiple types of DNA to be detected from each measured fluorescence intensity; a fifth step of identifying a microcompartment affected by one or more bubbles; and a sixth step of excluding data obtained for the microcompartment affected by one or more bubbles, from all data obtained for the plurality of microcompartments. In some embodiment, in the fifth step, the microcompartments, each having a number of peaks in a differential curve of a melting curve created from each fluorescence intensity measured in the third step, the number being greater than or equal to a predetermined number, may be identified as the one or more of that microcompartments affected by one or more bubbles. In some embodiment, if two or more adjacent of the microcompartments are identified as the microcompartment affected by one or more bubbles in the fifth step, data of the two or more adjacent of the microcompartments may be excluded in the sixth step; and if a single of the microcompartments is identified as the microcompartment affected by one or more bubbles in the fifth step, data of the single one may not be excluded in the sixth step. In the fifth step, the two or more adjacent of the microcompartments affected by one or more bubbles may be identified by image analysis of the microcompartments. In the fifth step, the two or more adjacent of the microcompartments affected by one or more bubbles may be identified by selecting the microcompartments, each having a number of peaks in a differential curve of each of the melting curves created from each of the fluorescence intensity measured in the third step, the number being greater than or equal to a predetermined number, followed by confirming whether the selected microcompartments are affected by one or more bubbles or not by image analysis of the microcompartments. In the fifth step, two or more adjacent of the microcompartments, each having a number of peaks in the differential curve, the number being greater than or equal to a predetermined number may be selected; and in the fifth step, a single one of the microcompartments having a number of peaks in the differential curve, the number being greater than or equal to a predetermined number may not be not selected. Furthermore, in the first step, the solution containing the multiple types of DNA may be subjected to limiting dilution. In the fourth step, the type of DNA to be detected may be identified in each of the microcompartments based on the melting temperature; and in the fifth step, the two or more of the microcompartments affected by one or more bubbles may be identified, further based on a predetermined reference melting temperature for each of the identified type of DNA. The microcompartments may be constituted by wells arranged in an array or droplets dispersed in oil. In the fourth step, the melting temperature may be calculated as an inflection point in the melting curve created from each fluorescence intensity measured in the third step.

In some embodiment, the present invention also provides a DNA detection system comprising: a first device having a plurality of microcompartments for comprising a DNA solution containing a fluorescent-labeled probe or a DNA intercalator; a second device for capturing an image of the first device; a third device for regulating temperature of the microcompartments in order to perform PCR in the microcompartments; a fourth device for making the imaging device capture an image to acquire fluorescence intensity in the microcompartments as temperature changes; and a fifth device for detecting generation of one or more bubbles from the acquired fluorescence intensity in the microcompartments.

### EFFECTS OF THE INVENTION

The invention provides a novel DNA detection system and DNA detection method, by which the microcompartments located in the vicinity of generated bubbles are detected and target genes are more accurately counted in digital PCR using melting curve analysis.

### BRIEF DESCRIPTION OF THE DRAWINGS

[FIG. 1] FIG. 1 shows schematic drawings illustrating a method for measuring the melting temperature of DNA with the fluorescent-labeled probe in the DNA detection method.
[FIG. 2] FIG. 2 shows schematic drawings illustrating the relations between droplets of a PCR solution and bubbles in a case that pits are used as the microcompartments to capture droplets of PCR solution.
[FIG. 3] FIG. 3 shows schematic drawings illustrating the relations between wells and bubbles in a case that wells are used as the microcompartments.
[FIG. 4] FIG. 4 shows drawings illustrating data regarding fluorescence intensity of a fluorescent dye contained in the wells under varying temperature and data regarding the melting temperature obtained from the change in the fluorescence intensity in each well under varying temperature.
[FIG. 5] FIG. 5 shows drawings illustrating measurement results from the DNA detection method.
[FIG. 6] FIG. 6 shows schematic diagrams illustrating a fluorescence measurement unit.
[FIG. 7] FIG. 7 shows a schematic diagram illustrating a digital PCR system.
[FIG. 8] FIG. 8 shows a drawing illustrating a database used for the DNA detection method.
[FIG. 9] FIG. 9 shows a flowchart of the method for measuring melting temperature using the devices illustrated in FIG. 6 and FIG. 7.
[FIG. 10] FIG. 10 shows drawings illustrating measurement results displayed on a monitor.
[FIG. 11] FIG. 11 shows drawings illustrating measurement results displayed on a monitor.
[FIG. 12] FIG. 12 shows drawings illustrating measurement results obtained in Example of the invention.

### DETAILED DESCRIPTION OF EMBODIMENTS

Objects, features, advantages, and ideas thereof of the present invention will be apparent to those skilled in the art from the description of the present specification. From the description of the present specification, those skilled in the art can easily reproduce the present invention. The embodiments, specific examples, and the like of the invention described below will illustrate preferred embodiments of the invention merely for the purpose of exemplification and explanation, to which the present invention is by no means limited. It will be apparent to those skilled in the art that various alterations and modifications can be made on the basis of the description herein, without departing from the spirit and scope of the invention disclosed herein.

### (1) DNA Detection Method

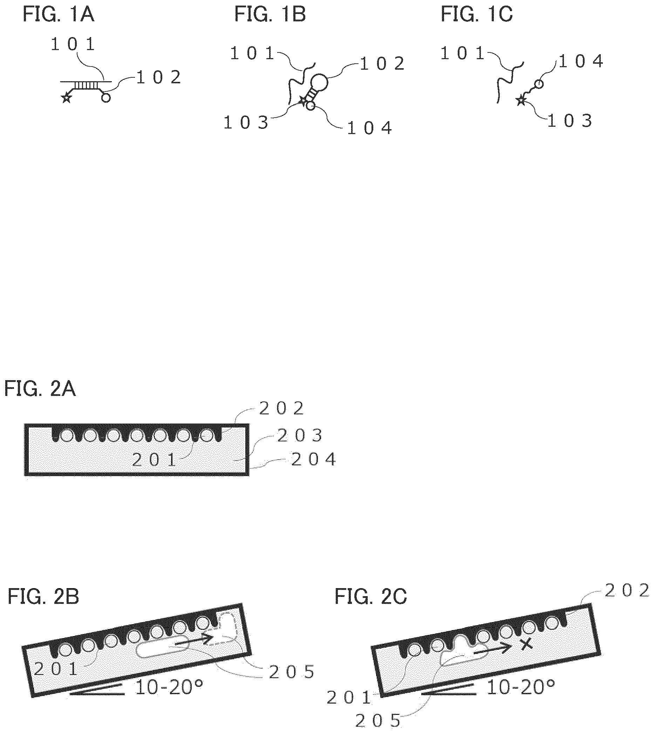

An embodiment in this specification relates to a DNA detection method comprising: a first step of dividing a DNA solution that contains a fluorescent-labeled probe or a DNA intercalator and multiple types of DNA to be detected into a plurality of aliquots; a second step of performing PCR in microcompartments, each containing one of aliquots; a third step of measuring fluorescence intensity from the fluorescent-labeled probe or the DNA intercalator in each of the microcompartments as temperature changes; a fourth step of calculating a melting temperature of the multiple types of DNA to be detected from each measured fluorescence intensity; a fifth step of identifying a microcompartment affected by one or more bubbles; and a sixth step of excluding data obtained for the microcompartment affected by one of more bubbles from all data obtained for the plurality of microcompartments. This DNA detection method is described in detail below with reference to the schematic diagrams of FIGS. 1 to 7.

### (First Step)

In this step, the specimen solution containing the fluorescent-labeled probe or the DNA intercalator and multiple types of DNA to be detected is divided into the plurality of aliquots.

The first step is to prepare the specimen solution containing DNA derived from a biological sample. The specimen solution may contain multiple types of target gene. The specimen solution is added to a PCR solution. The PCR solution contains DNA polymerases, primers, DNA intercalators or fluorescent-labeled probes, deoxyribonucleotides, and buffer solution. In this process, the specimen solution comes to contain the fluorescent-labeled probe or the DNA intercalator. Molecular beacons designed with a structure that can hybridize to the target gene are preferably used as a fluorescent-labeled probe.

The specimen solution is microfractionated into microcompartments. When microfractionated, the specimen solution is preferably subjected to limiting dilution such that no or one DNA molecule is contained in each microcompartment.

The biological sample to be used is not limited to any particular sample as long as it contains the DNA to be detected. Examples of biological samples include individual samples (such as body fluid, tissue, cells or excretions of animals or plants) or soil samples (containing fungi or bacteria). The body fluid is exemplified by blood, saliva, and cerebrospinal fluid. The blood contains cell-free DNA (cfDNA), or circulating tumor DNA (ctDNA). The tissue is exemplified by disease-affected portions collected by surgical operation or biopsy (e.g., cancer tissue from breast and liver, etc.). The tissue may be a fixed tissue, such as a formalin-fixed paraffin-embedded tissue section (FFPE). The cells are exemplified by those collected by biopsy (from the affected portion or nearby) and circulating tumor cells circulating in blood.

Pretreatmemt of these specimens is not limited, and after collection from a living body or circumstances, they may be added to a suspension liquid and homogenized or may be solubilizd in a solubilizing liquid; then they may be used without further processing. It is more preferable to use nucleic acid that have been extracted and purified from the specimens.

### (Second Step)

In this step, PCR is performed in each microcompartment containing the aliquot. PCR may be preferably so-called normal PCR, more preferably digital PCR.

### (Third Step)

In this step, the fluorescence intensity of the fluorescent-labeled probe or the DNA intercalator is measured in each microcompartment as temperature changes. It may be measured as temperature changes during PCR or while heating of the specimen solution independently from PCR (for example, after completion of PCR). The fluorescent-labeled probe or the DNA intercalator used in this measuring step may be the same or different from the fluorescent-labeled probe or the DNA intercalator used for PCR.

FIG. 1 shows schematic drawings illustrating behaviors of a fluorescent-labeled probe under varying temperature of the reaction solution. The fluorescent-labeled probe may be exemplified by molecular beacons and Taqman probes. FIG. 1 is illustrated using a molecular beacon as an example. Molecular beacons are constructed as oligonucleotides and have sequences that are complementary to the sequence between primer pairs used in PCR to amplify the target gene. Both ends of the molecular beacon have sequences which are complementary to each other, and one end is bound to fluorescent dye 103 and the other end is bound to a quencher dye (quencher) 104. In the initial stage of PCR, the molecular beacon 102 is in a free state as illustrated in FIG. 1B. The molecular beacon 102 in this state forms a stemmed loop and does not emit fluorescence since the fluorescent dye 103 and the quencher 104 are positioned nearby. When heating the specimen solution in the first denaturation step, the beacon comes to have a more flexible structure as illustrated in FIG. 1C, but the fluorescence remains quenched since the fluorescent dye and the quenching dye are not constantly kept apart. In an annealing step, when the temperature is lowered to about room temperature, a loop moiety of the molecular beacon 102 anneals to amplified DNA 101 in the specimen solution as illustrated in FIG. 1A. This causes the fluorescent-labeled probe 102 emits strong fluorescence because the fluorescent dye 103 and the quencher 104 are constantly kept apart. In the next elongation step, the molecular beacon 102 returns to the free state as illustrated in FIG. 1B and fluorescence is quenched. In the next denaturation step, the beacon again takes the form as illustrated in FIG. 1C and fluorescence remains quenched. Since these processes are repeated in the PCR, it suffices to measure the fluorescence intensity at some point during heating or cooling. The fluorescence intensity may be measured in the same way after completion of PCR. In a case that a DNA intercalator is used, the DNA intercalator is intercalated between two strands of sample DNA and emits fluorescence when it is double-stranded, while it is released from the sample DNA and it quenches when it is single-stranded. Hence, it suffices to measure the fluorescence intensity at some point during heating or cooling in PCR like molecular beacon 102. After completion of PCR, the fluorescence intensity may be measured by heating or cooling only to measure the fluorescence intensity.

The combination of the fluorescent dye 103 and the quencher 104 of molecular beacon 102 used in the invention is not particularly limited as long as they are commonly combined for real-time PCR. The fluorescent dye 103 is exemplified by FAM, VIC, ROX, Cy3, and Cy5. The quencher 104 is exemplified by TAMRA, BHQ1, BHQ2, and BHQ3.

In a case that two types of the target genes having different sequences are used, they are detectable in a single reaction system by preparing the molecular beacons 102 with sequences specific to each of the target genes and with different fluorescent dyes bound thereto.

The DNA intercalator is not particularly limited as long as the fluorescence intensity increase by binding to double-stranded DNA and it is applicable to detection of the double-stranded DNA. Specific examples applicable here include SYBR^{®} (registered trademark) Green I, SYBR Gold, PicoGreen^{®} (registered trademark), SYTO^{®} (registered trademark) Blue, SYTO Green, SYTO Orange, SYTO Red, POPO^{®} (registered trademark)-1, BOBO^{®} (registered trademark)-1, YOYO^{®} (registered trademark)-!, TOTO^{®} (registered trademark)-!, JOJO^{®} (registered trademark)-1, POPO-3, LOLO^{®} (registered trademark)-1, BOBO-3, YOYO-3, TOTO-3, PO-Pro^{®} (registered trademark)-1, YO-Pro^{®} (registered trademark)-!, TO-Pro^{®} (registered trademark)-!, JO-Pro^{®} (registered trademark)-1, PO-Pro-3, YO-Pro-3, TO-Pro-3, TO-Pro-5, and ethidium bromide, etc. The DNA intercalator, if being heat resistant, may be added to the reaction solution in advance of PCR.

### (Fourth Step)

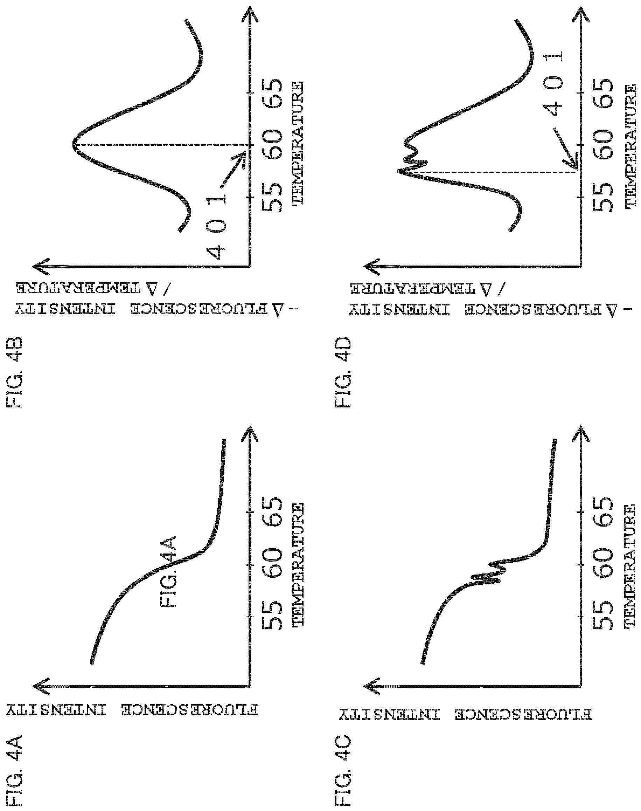

In this step, the Tm of the DNA to be detected is calculated from each measured fluorescence intensity. A method of calculating the melting temperature is not particularly limited. One possible method may be such as acquiring a function of the fluorescence intensity and the temperature, the derivative of the function is then obtained, and the temperature at which the derivative becomes its maximum may be calculated and used as the Tm. Alternatively, a function of the fluorescence intensity and the temperature may be plotted on a graph to create a melting curve (that is, a curve representing changes in the fluorescence intensity in response to temperature change), the fluorescence intensity is further differentiated with temperature to create a differential curve, and the temperature corresponding to the peak may be determined as the Tm. An exemplary melting curve is illustrated in FIG. 4A, and the differential curve obtained from this melting curve is illustrated in FIG. 4B. The temperature that gives an inflection point on the melting curve corresponding to the peak of the differential curve is calculated as Tm 401 of the double-stranded DNA. The Tm of the fluorescent-labeled probe used for detecting the target gene is adjustable according to known techniques when designing fluorescent-labeled probes. For example, it is typically adjustable by changing the sequence or chain length of the probe. It is alternatively adjustable by utilizing artificial DNAs such as peptide nucleic acid (PNA) or locked nucleic acid (LNA).

### (Fifth Step)

In this step, the microcompartment affected by bubbles are identified from the Tm calculated in the fourth step.

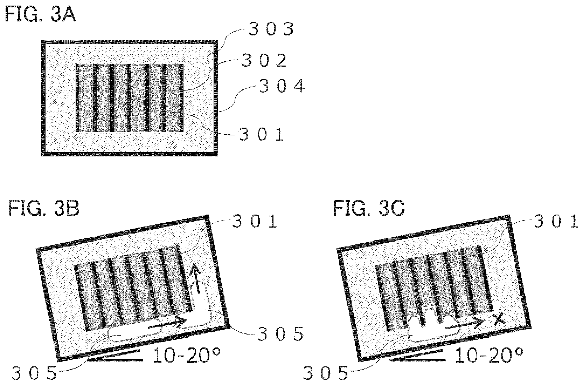

Exemple of the bubbles that formed near the microcompartments in the digital PCR using melting curve analysis are illustrated in FIG. 2 and 3.

A cartridge 204 illustrated in FIG. 2 has pits as the microcompartments for capturing droplets of the PCR solution. As illustrated in FIG. 2A, the inside of the cartridge 204 is filled with oil 203, and the droplets 201 are captured in droplet capturing pits 202 arrayed in the cartridge 204. In the digital PCR using melting curve analysis, fluorescence images of the droplets are acquired as temperature changes and the melting curve analysis is performed for each droplet. During this process, a bubble 205 may generate in the cartridge 204, with high probability of generation particularly at high temperatures. The presence of the bubble 205 under the droplets 201 will cause noise in the measurement of fluorescence intensity, and therefore it is needed to expel bubbles from beneath the droplets 201, by tilting the cartridge 204 as illustrated in FIG. 2B. However, in a case where the droplets 201 are not evenly captured in the droplet capturing pits 202 as illustrated in FIG. 2C, the bubble 205 would occasionally be captured in a vacant droplet capturing pit 202 even if the cartridge 204 is tilted, and it may possibly result in an incorrect measurement of the fluorescence intensity at the droplet 201 near the bubble.

A cartridge 304 illustrated in FIG. 3 has wells as the microcompartments for containing the PCR solution. As illustrated in FIG. 3A, the inside of the cartridge 304 is filled with oil 303. PCR solution 301 is added to the wells of a through hole chip 302 in a cartridge 304. Like the droplets, when the wells are used, a bubble 305 can be generated in the cartridge 304 with high probability of generation particularly at high temperatures. The presence of the bubble 305 under the through hole chip 302 will cause noise in the measurement of fluorescence intensity, and therefore it is needed to expel bubbles from beneath the through hole chip 302, by tilting the cartridge 304 as illustrated in FIG. 3B. However, in a case where the PCR solution is not evenly added to the through hole chip 302 as illustrated in FIG. 3C, the bubble 305 would occasionally be captured in the wells with less volume of PCR solution even if the cartridge 304 is tilted, and it may possibly result in the incorrect measurement of the fluorescence intensity at the wells near the bubble.

FIG. 4 illustrates exemplary melting curves from the microcompartments when the bubbles were not generated and when bubbles were generated therein. In a case when bubble were not generated, the measurement of the changes in the fluorescence intensity in the microcompartments as temperature change resulted in a smooth melting curve as illustrated in FIG. 4A. A differential curve derived therefrom had a single peak as illustrated in FIG. 4B, and a peak temperature was the Tm 401. In contrast, in a case when bubbles were generated, the measurement of the changes in the fluorescence intensity in the microcompartments as temperature changes demonstrated the fluctuation of fluorescence intensity with the movement of the bubble as illustrated in FIG. 4C. Therefore, the differential curve have a plurality of peaks as illustrated in FIG. 4D, and the melting temperature of the target gene in the microcompartments may not be identified, or the melting temperature may be incorrect.

### (Sixth Step)

In this step, data from the microcompartment affected by the bubbles is excluded from all data obtained for the plurality of microcompartments.

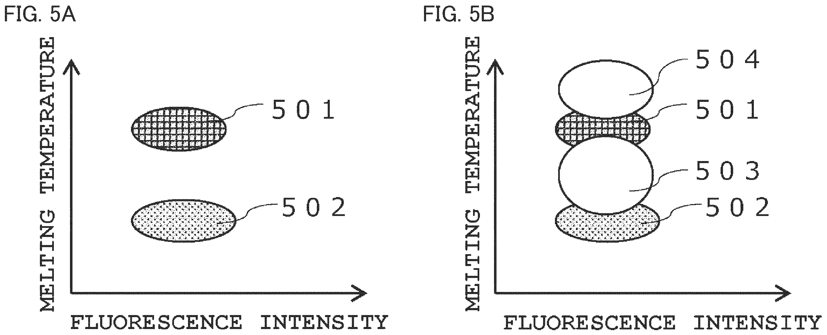

FIG. 5 illustrates exemplary results of the melting curve analysis for the microcompartments when bubbles are not generated and when bubbles are generated therein. As illustrated in FIG. 5A, in the microcompartment that contains a target gene having a wild-type allele, a Tm 501 that corresponds to the fluorescent-labeled probe of such wild-type allele is detected, meanwhile in the microcompartment that contains a target gene having a mutant allele, a Tm 502 that corresponds to the fluorescent-labeled probe of such mutant allele is detected. However, when the bubbles are generated therein, the microcompartment that contains target genes having wild-type alleles may erroneously show melting temperatures 503 and 504 that are different from the melting temperature of the wild-type allele, as illustrated in FIG. 5B. This results in overlapping of distribution of the melting temperature 503, which is different from the melting temperature of the wild-type allele, with distribution of the melting temperature 502 of the mutant allele, and the microcompartment containing the target gene having the wild-type allele may be identified as the microcompartment containing the target gene having the mutant allele and *vice versa.* Detection of the generation of the bubbles and determination of the position of the microcompartment near the bubbles can exclude the microcompartment that demonstrates the Tm 503 which differs from the Tm of the wild-type allele from analytical data. This reduces erroneous determination of the gene in the microcompartments and enables more accurate gene detection.

### (2) DNA Detection System

The DNA detection system disclosed herein is intended to detecting target genes in DNA solution, includes: a first device having a plurality of microcompartments for comprising the DNA solution that contains a fluorescent-labeled probe or a DNA intercalator; a second device for capturing an image of the first device; a third device for regulating temperature of the microcompartments in order to perform PCR in the microcompartments; a fourth device for makeing the imaging device capture an image to acquire fluorescence intensity in the microcompartments as temperature changes; and a fifth device for detecting generation of one or more bubbles from the acquired fluorescence intensity in the microcompartments. This DNA detection system executes the aforementioned DNA detection method.

In addition to the aforementioned devices, the DNA detection system may further include a sixth device for measuring the intensity of fluorescence emitted from the DNA solution; a computer for calculating the Tm of the double-stranded DNA based on the melting curve representing changes in the fluorescence intensity as temperature of the DNA solution changes; and a monitor for displaying data transmitted from the computer. All of these devices may physically be in a single device; each may exist stand alone; or some of these devices may be combined to form a single device, while the others may exist stand alone.

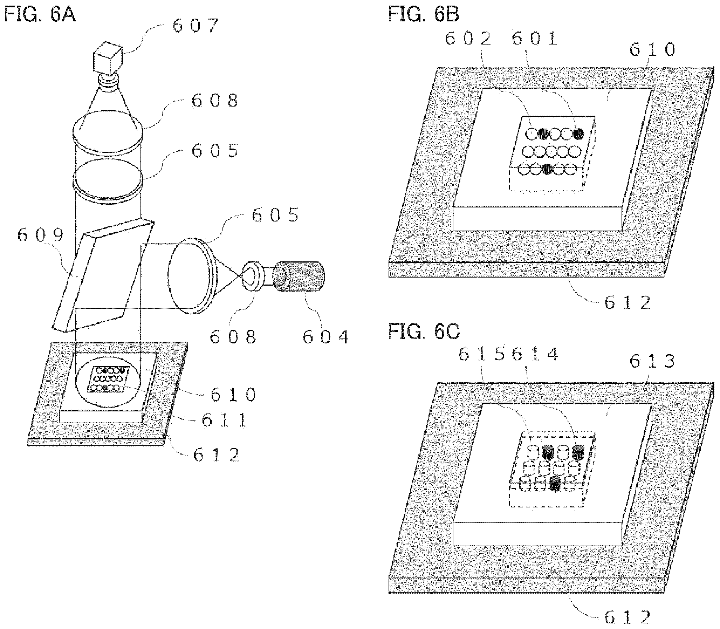

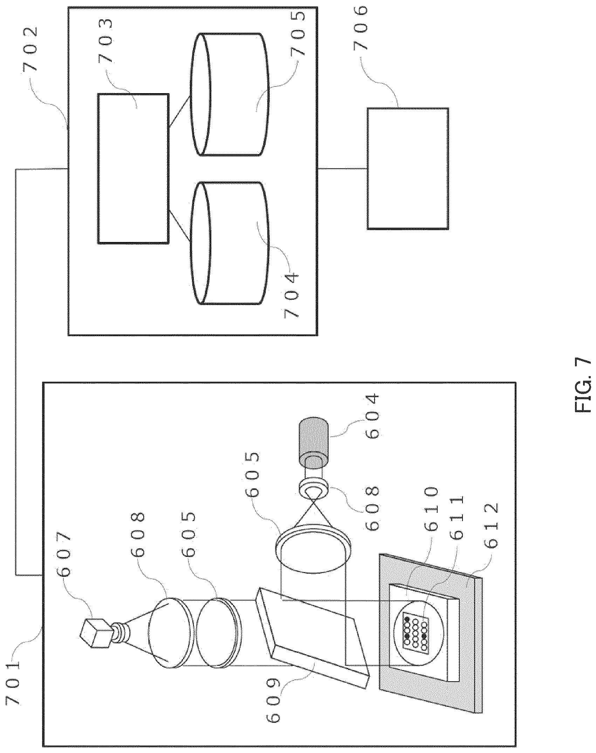

The DNA solution may be contained by any type of carrier, such as droplets in the well in the plate or the cartridge as shown in FIG. 2, or may be contained in the wells in the plate or the cartridge as shown in FIG. 3. As an example of DNA detection system, FIGS. 6 and 7 show a DNA detection system with a fluorescence measurement unit.

FIG. 6 is a schematic diagram of the fluorescence measurement unit for measuring the fluorescence intensity emitted from the DNA solution. FIG. 7 is a schematic diagram of the digital PCR system. The fluorescence measurement unit in FIG. 6 measures a color and fluorescence intensity of the fluorescent dye contained in the droplet or the well subjected to limiting dilution. The digital PCR system in FIG. 7 includes the fluorescence measurement unit illustrated in FIG. 6, a computer for calculating the melting temperature of double-stranded DNA and analyzing the measurement data, and the monitor for displaying the result.

The fluorescence measurement unit illustrated in FIG. 6A includes a light source 604, a fluorescence filter 605, and a detection unit such as a photomultiplier and a camera 607. The fluorescence measurement unit may have multiple light sources and/or multiple detection units for each color of the fluorescent dye; or a single light source may excite multiple fluorescent dyes having different excitation wavelengths through multiple fluorescent filters605, and a single detection unit may simultaneously detect multiple fluorescence having different wavelengths through multiple fluorescent filters 605.

For example, as illustrated in FIG. 6B, the plurality of droplets 611 are arranged in an array in a droplet detection cartridge 610 as illustrated in FIG. 2, and set the droplet detection cartridge 610 on a temperature control stage 612 which is a temperature control unit. The temperature control stage 612 changes the temperature of each compartments in order to perform PCR in each compartment. The temperature of the droplet detection cartridge is changed on the temperature control stage 612 to measure the changes in the fluorescence intensity of the droplets as temperature changes. The fluorescence intensity changes differently for the droplets 601 which contains the target gene and for the droplets 602 which does not contain the target gene.

Measurement procedure is, for example, as follows. First, irradiate each droplet 611 with excitation light from the light source 604 through a lens 608, the fluorescence filter 605, and a dichroic mirror 609. The excitation light excites the fluorescent substance contained in each droplet 611, and a CCD camera 607 detects the emitted fluorescence through the dichroic mirror 609, the fluorescence filter 605, and the lens 608. The CCD camera 607 is an example of the imaging device.

Alternatively, a well-type detection cartridge 613 as shown in FIG. 3 may also be used as illustrated in FIG. 6C. A reaction solution containing specimens is added to the wells of the well-type detection cartridge 613. PCR is then performed in the wells, and the cartridge is set the well-type detection cartridge 613 on the temperature control stage 612 which is the temperature control unit. The temperature of the well-type detection cartridge 613 is changed by the temperature control stage 612 and the changes in the fluorescence intensity in the wells are measured as temperature changes. The fluorescence intensity changes differently for the wells 614 which contains the target gene and for the wells 615 which does not contain the target gene.

Measurement procedure is, for example, as follows. First, irradiate each well with excitation light from the light source 604 through the lens 608, the fluorescence filter 605, and the dichroic mirror 609. The excitation light excites the fluorescent substance contained in each well, and a CCD camera 607 detects the emitted fluorescence through the dichroic mirror 609, the fluorescence filter 605, and the lens 608. When wells are used as in FIG. 6D, PCR to the melting curve analysis can be performed in the cartridge 613 without arranging the droplets in the droplet detection cartridge.

For observation of the changes in the fluorescence intensity in the microcompartments as temperature changes, a tilt adjustment unit (not shown) may be provided under the temperature control stage 612. The tilt adjustment unit removes the bubbles generated in the cartridge due to heating by the temperature control stage 612. This prevents bubbles from interfering with the acquisition of fluorescent images when measuring the fluorescence intensity in each well while lowering the temperature of the sample with the temperature control stage 612.

As illustrated in FIG. 7, the fluorescence data detected by the fluorescence measurement unit 701 is sent to a computer 702. In the computer 702, the Tm of the amplicon is calculated by an analysis unit 703, and the data are stored in a memory 705. The memory 705 stores the Tm of the double-stranded DNA in each microcompartment. As described later, the melting temperature is obtained based on the changes in the fluorescence intensity in the image captured by the imaging device as temperature changes as described below.

The relationship between the type of gene and the melting temperature is prepared in advance in a database 704. The database 704 stores information representing predetermined melting temperatures as reference (hereinafter referred to as reference Tm) for each of wild-type and mutant of target gene. Referring to the database 704, the genotype of the target gene is identified based on the measured melting temperature in the memory 705. The number of microcompartments identified is then counted for each genotype.

A monitor 706 displays measurement results. The monitor 706 is an example of an output device and the display process on the monitor 706 may be replaced by an output process to another output device (printer, non-volatile memory device, etc.).

The DNA detection system disclosed herein may include a sample dividing device. The sample dividing device prepares a limiting dilution of the DNA solution containing the target gene and divides it into aliquots.

The DNA detection system may also include an amplification device to amplify DNA in the microcompartments.

### (3) DNA Detection Method using DNA Detection System

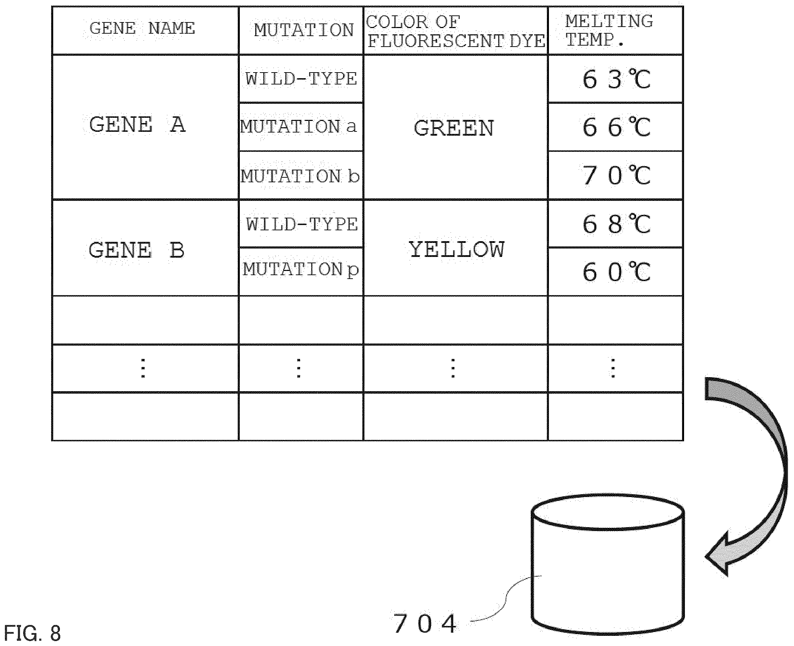

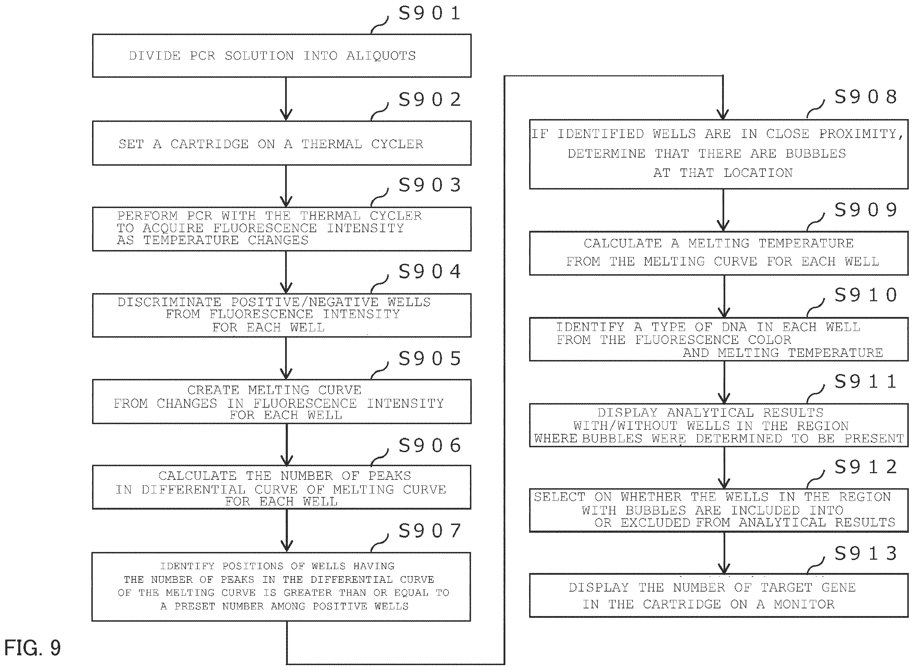

An embodiment of a method for detecting DNA using aforementioned DNA detection system is described below with reference to FIGS. 8 and 9. In this embodiment, changes in the fluorescence intensity in PCR are used to identify the Tm of DNA. The melting curve and the differential curve are also used to identify the Tm.

FIG. 8 illustrates an example of a database containing data representing the reference Tm of genes prepared prior to the measurement of digital PCR. The data shown in FIG. 8 can be measured in advance by pilot experiments and stored as the database 704. In the example in FIG. 8, the memory stores the reference Tm data for the wild-type and mutant of each gene as a database. In this example, the reference Tm correspond to each genotype. Multiple mutants may be defined for a single gene. The reference Tm may be identified as a value representing a single temperature, as in this example, or as a range of temperature. In addition to the reference Tm, the memory also stores data indicating the color of the fluorescent dye for each genotype of each gene in this example.

First, a DNA solution containing the fluorescent-labeled probe or the DNA intercalator and the plurality of types of DNA to be detected is divided into the plurality of microcompartments in the cartridge 613 (S901). In this embodiment, the wells are used as the microcompartments.

To prevent the DNA solution divided into the wells from vaporizing during PCR and the melting curve analysis measurement, oil may be added to the top face of the DNA solution. The oil may preferably a chemically inert substance that is insoluble or poorly soluble in the PCR solution and stable to temperature change at high temperatures such as PCR. The oil can be used here includes fluorinated oil, silicone-based oil, and hydrocarbon-based oil. The fluorinated oil is exemplified by perfluorocarbon and hydrofluoroether. The fluorinated oil with long carbon chains are preferred because of the low volatility. The silicone-based oil is exemplified by polyphenylmethylsiloxane and trimethylsiloxysilicate. The hydrocarbon-based oil is exemplified by mineral oil, liquid paraffin, and hexadecane. The oil may be used with surfactants added thereto. Type of the surfactant is not particularly limited. Surfactants such as Tween 20, Tween 80, Span 80, and Triton X -100 are applicable.

Next, set the cartridge 613 on a thermal cycler (S902).

PCR is performed in each wells by the thermal cycler (S903). DNA is amplified by repeating a cycle that includes the denaturation step, the annealing step, and the elongation step. The DNA intercalator intercalates into the amplified DNA, meanwhile the molecular beacon hybridizes to the amplified DNA. This increases the fluorescence intensity as DNA is amplified. The person skilled in the art can easily set the reaction conditions including temperature, time, and number of cycles for each steps. After completion of PCR, by lowering the temperature to room temperature, the amplified DNA forms double strands.

In this process, changes in the fluorescence intensity are measured for each well as temperature changes by the thermal cycler (S903). Specifically, the cartridge 613 is placed on the temperature control stage 612 of the DNA detection system. While the temperature of the cartridge 613 is changed by the temperature control stage 612, the fluorescence measurement unit 701 measures the fluorescence intensity from the fluorescent-labeled probe or the DNA intercalator in each well. The fluorescence intensity may be directly measured with the photomultiplier 606 or may be acquired by capturing fluorescence images and by analyzing the images. More specifically, for example, the computer 702 or other component functions as an imaging control unit causes the imaging device to capture images. The obtained fluorescence images are sent to the computer 702, then the analysis unit 703 calculates the fluorescence intensity from the fluorescent images for the each microcompartment. The obtained fluorescence intensity data for each microcompartment are stored in the memory 705.

From the obtained fluorescence intensity data for each microcompartment, positive wells which contain the target gene and negative wells which does not contain the target gene are discriminated (S904).

Meanwhile, the analysis unit 703 creates a melting curve based on the fluorescence intensity data (S905) and calculates a number of peaks of the differential curve (S906). The positions of the wells having a preset number or more of peaks in the differential curve of the melting curve are identified among the positive wells (S907). If the positions of wells having the preset number or more of peaks in the differential curve of the melting curve are in close proximity, the positions are determined that the bubbles are present at that location (S908). Round bubbles may be detected by image recognition using the entire fluorescence images of the microcompartments and may be used as information to detect bubbles. The Tm is calculated from the melting curve for each well and then stored in the memory 705 (S909). The Tm calculated based on the measurement of the fluorescence intensity is called "measured Tm" and is distinguished from the predetermined reference Tm. Referring to the database 704 in the memory 705, the type of DNA in the wells is then determined by referring to the reference Tm based on the color of fluorescence and the measured Tm (S910).

Analytical results are displayed, including graphs of the relationship between the fluorescence intensity and the melting temperature, including and excluding the wells in the region where are determined that the bubbles are present at that location (S911). User chooses to include or exclude the wells in the bubble region from the analytical results (S912).

Lastly, A numbers of the target gene in the cartridge is displayed on the monitor (S913). An specific display example is shown below.

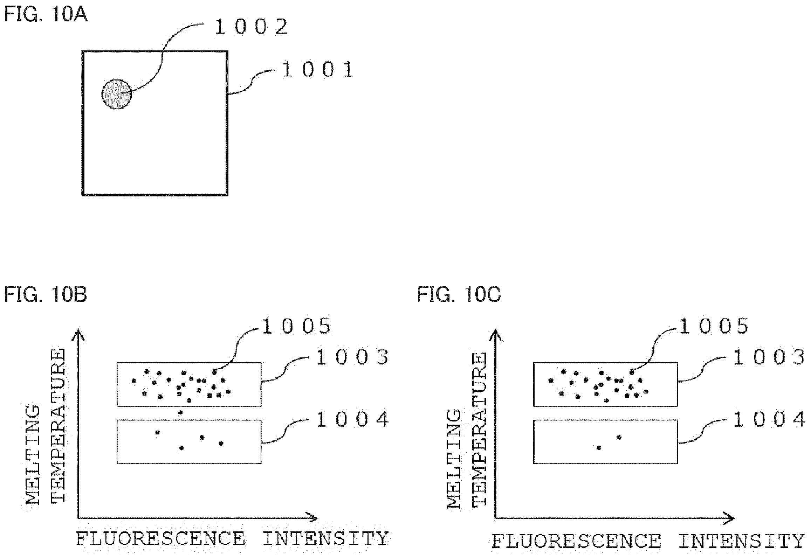

As illustrated in FIG. 10A, a mark 1002 indicating the estimated bubble position may be superimposed on an illustration or a fluorescence image of a chip 1001 having microwells in the cartridge. The monitor display, including the position of the microcompartments near bubbles and the number of such compartments, as described above, can be used for accuracy management of the digital PCR. In addition, the graph of fluorescence intensity versus melting temperature may be overlayed the melting temperature range 1003 for the wild-type and 1004 for the mutant, and the plots 1005 of each microcompartments calculated by the melting curve analysis. The analysis results may be displayed including (FIG. 10B) and excluding (FIG. 10C) the wells in the region where are determined that the bubbles are present. This allows the user can use both results.

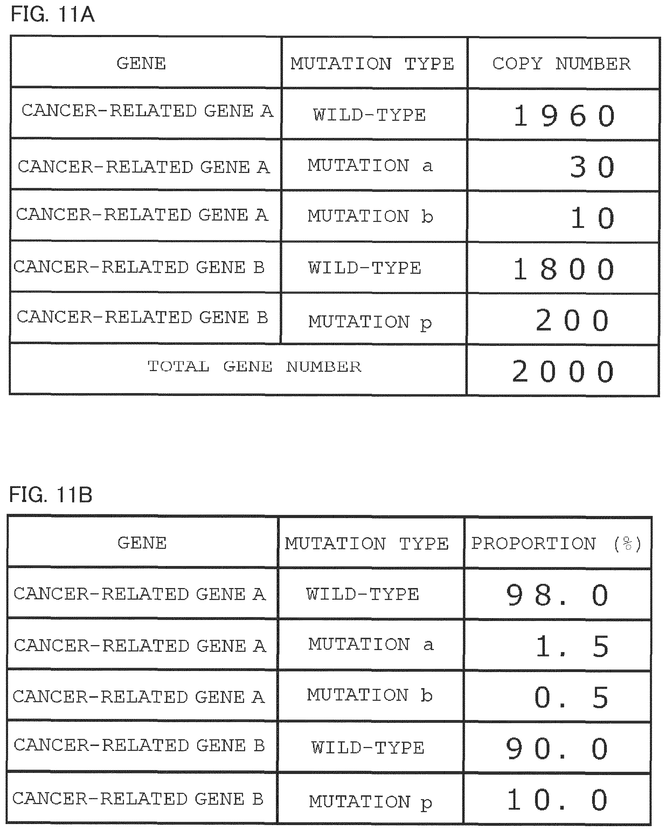

FIG. 11 illustrates examples of measurement results displayed on the monitor. These examples represent the results of measuring the wild-types and mutants of oncogenes A and B as target genes. The number of DNA in the specimen solution may be displayed for each type of oncogenes and mutations after selecting one of the analysis results, including or excluding the wells in the region determined to contain bubbles, as illustrated in FIG. 11A. The total number of genes may be displayed as calculated from the sum of the wild-type and the mutant of the target gene. This type of display allows the user of the device to easily understand the contents of the specimen solution. Alternatively, as illustrated in FIG. 11B, a proportion of the counted DNA in the specimen solution may be displayed for each type of oncogene and mutation. The example of FIG. 11B represents proportions of mutated genes in the target gene.

The result displayed on the monitor may be the number or proportion of genes in the specimen solution as illustrated in FIG. 11, or graphs plotting the measured values of the specimen solution on a two-axis coordinate of the fluorescence intensity of the fluorescent-labeled probe versus the measured Tm as illustrated in FIG. 10, or may contain both. The display may alternatively contain a histogram plotting the number of DNA in the specimen solution against the fluorescence intensity of the fluorescent-labeled probe or the measured Tm.

A Range of the fluorescence intensity of the fluorescent-labeled probe used to count the number of DNA may be arbitrarily changed by the user. A range of the reference Tm used to the number of DNA may also be arbitrarily changed by the user. The DNA detection system may accept operation for changing these ranges and may change the relevant ranges. This enables the user to look at the graph or histogram of the measurement results, change the ranges of the fluorescence intensity and/or the reference Tm, and re-count the number of DNA in the specimen solution within a new range.

Since the specimen solutions are treated as solution in the wells or the droplets as described above, the number of DNA in the specimen solution may be replaced with the number of wells or the number of droplets.

If the number of microcompartments near the bubbles exceeded a predetermined number, an alert may be displayed on the monitor 706 as a measurement error. The alert informs the user that the digital PCR system needs adjustments.

### (4) Method for Detecting the Plurality of Target Genes

The digital PCR using melting curve analysis can discriminate the plurality of target genes by utilizing the fact that the Tm of the fluorescent-labeled probe and DNA differs among each target gene. The case in which the plurality of wild-type and mutant alleles are included as target genes can be exemplified, but not particularly limited thereto.

For example, in a case where target gene P and target gene Q are contained in the DNA solution to be analyzed, the fluorescent-labeled probe corresponding to the target gene P hybridizes to the DNA amplified by PCR and emits fluorescence in the microcompartments containing the target gene P. From analysis of the emitted fluorescence, the Tm of the target gene P corresponding to the fluorescent-labeled probe can be calculated. Meanwhile, the fluorescent-labeled probe corresponding to the target gene Q hybridizes to the DNA amplified by PCR and emits fluorescence in the microcompartments containing the target gene Q. From analysis of the emitted fluorescence, the Tm of the target gene Q corresponding to the fluorescent-labeled probe can be calculated. Based on the fluorescence intensity, the type of fluorescence (e.g., color), and the melting temperature, presence or absence of the target gene P and target gene Q may be determined.

Since the Tm of DNA is not affected by the reaction efficiency of PCR or variation of the fluorescence measurement in plane, the Tm of DNA can be used to determine the genotype of DNA in microcompartments with high precision. For example, fluorescence intensity changes as temperature changes are measured for DNA in microcompartments using fluorescent-labeled probes having sequences with different Tms for each target gene. Genotyping of DNA in microcompartments is possible by performing melting curve analysis from the measurement and comparing Tms.

When the plurality of target genes are detected simultaneously, the discrimination between genes can be based on the reference Tms and the measured Tms, and is preferably based on the range of reference Tm. For example, if the measured Tm for a certain well is within a predetermined range(for example, within ± 1°C of the reference Tm), including the reference Tm of a certain target gene stored in the database 704, the certain well is then determined to contain the certain target gene. Using such the range of the reference Tm enables more accurate identification with proper consideration of an allowable range.

Alternatively, the ratio or difference of the fluorescence intensity measured at different temperatures may be used as data of the fluorescence intensity. For example, the fluorescence intensity can be standardized by using the ratio or difference between the fluorescence intensity at a temperature lower than the reference Tm and that at a temperature higher than the reference Tm. For example, if this ratio or difference for a certain well is within the predetermined range, the certain well is determined to be positive; otherwise, the certain well is determined to be negative.

For example, influence of the fluorescent-labeled probe itself, i.e., influence of the background, can be eliminated by subtracting the fluorescence intensity at 85°C from the fluorescence intensity at 50°C.

Methods for determining the range of the fluorescence intensity and the reference Tm can be arbitrarily selected. For example, the ranges may be determined statistically by an operator based on the results of preliminary experiments, or it may be determined automatically by the DNA detection system. For every run of the digital PCR measurement, a threshold of the fluorescent intensity and the predetermined range of the reference Tm may be statistically determined using the measured data for each well in the cartridge.

Data for statistical identification of DNA in the wells may include any or all of the following items, or may include items other than these.
· Fluorescence intensity at a temperature lower than the reference Tm
· Fluorescence intensity at a temperature higher than the reference Tm
· Ratio of the fluorescence intensity at a temperature lower than the reference Tm to the fluorescence intensity at a temperature higher than the reference Tm
· Difference between the fluorescence intensity at a temperature lower than the reference Tm and the fluorescence intensity at a temperature higher than the reference Tm
· Features representing the reference Tm
· Features representing shape of the melting curve

### (5) Non-transitory computer-readable media

An embodiment of the present disclosure is a non-transitory computer-readable medium programmed to execute DNA detection method by DNA detection system. Another embodiment of the present invention is a non-transitory computer-readable medium storing the above program.

### EXAMPLES

This Example shows results of identification between *KRAS* gene and its mutants G12A and G13D by measuring the Tms of them in the wells using fluorescent-labeled probes.

First, genomic DNAs of wild-type, G12A and G13D mutants of *KRAS* gene (final concentration: 133 molecules/µL) were prepared. To prepare a PCR solution, the following essentials for PCR were added to the genomic DNAs: forward primer (final concentration: 0.25 µM), reverse primer (final concentration: 2.0 µM), fluorescent-labeled probe for wild-type (final concentration: 0.5 µM), fluorescent-labeled probe for G12A mutant (final concentration: 0.5 µM), and a 1x master mix (containing DNA polymerase and dNTP). The primer pairs were added at asymmetric concentrations to achieve overamplification of the complementary DNA strand of the fluorescent-labeled probes. The G13D mutant was detected using fluorescent-labeled probes for wild-type including a mismatched base contained therein. The sequences of the primers and the probes are as follows. Both fluorescent-labeled probes have complementary sequences near each end and are designed to form intramolecular duplex. Each probe also has HEX as a fluorescent dye bound to the 5' end and BHQ-1 as a quencher bound to the 3' end.
Forward primer: 5'- GTCACATTTTCATTATTTTTATTATAAGG -3' (SEQ ID NO: 1)
Reverse primer: 5'- GTATCGTCAAGGCACTCTTGCC-3' (SEQ ID NO: 2)
Fluorescent-labeled probe for wild-type: 5'-TTGGAGCTGGTGGCGT-3' (SEQ ID NO: 3)
Fluorescent-labeled probe for mutant: 5'-TTGGAGCTGCTGGCGT-3' (SEQ ID NO: 4)

To ensure that each well would contain one or none of the wild type or G12A or G13D mutant of *KRAS* gene, 15 µL of the PCR solution was added to each well. The DNAs were then amplified by PCR. The PCR reactions were performed by treatment at 96°C for 10 minutes, followed by 59 cycles of 60°C for 2 min and 98°C for 30 seconds, and finally 60°C for 2 minutes. After the reaction, the chip including the wells was observed the fluorescence intensity of each well while cooled from 85°C to 50°C on the temperature control stage, and the melting curves were measure and analyzed.

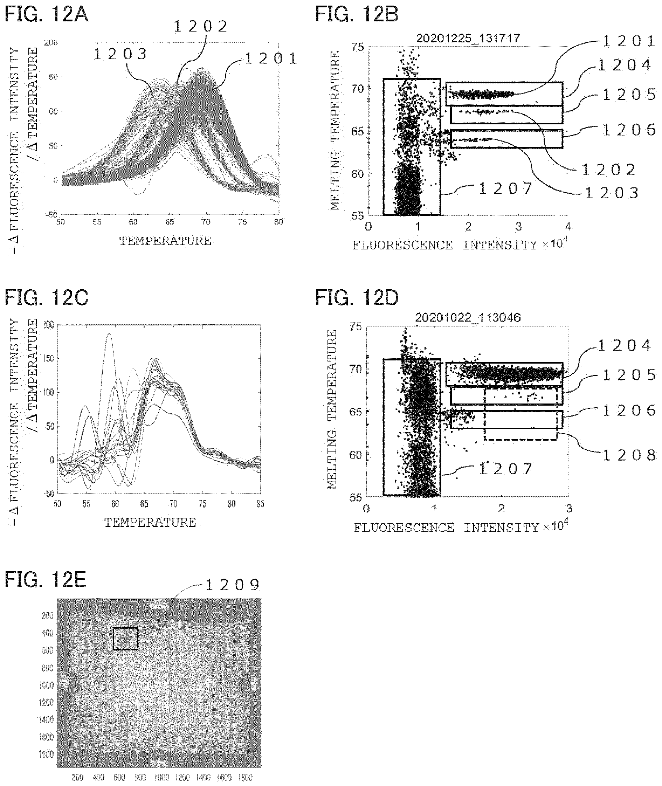

FIG. 12A shows differential curves of the melting curves for each well for a sample containing a mixture of *KRAS* genes: wild-type, G12A, and G13D mutants. The differential curve of the melting curve from each well had a single peak, and the temperature at the position of peak was calculated as the Tm. FIG. 12B is a graph based on the results in FIG. 12A, with the fluorescence intensity at 50°C for each well plotted on the abscissa and the melting temperature plotted on the ordinate. Excluding negative wells 1207, the positive wells were divided into three distributions based on differences in the Tms: a population 1201 with distribution around 69°C corresponds to the wells containing only the wild-type, a population 1202 with distribution around 66°C corresponds to the wells containing only the G12A mutant, and a population 1203 with distribution around 63°C corresponds to the wells containing only the G13D mutant. From the results, the Tm range for the wild-type 1204, the Tm range for the G12A mutant 1205, and the Tm range for the G13D mutant 1206 were determined.

When samples containing only the wild type of the *KRAS* gene were measured, a population 1208 with Tms outside the Tm range for the wild-type was observed as shown in FIG. 12D. The melting curve of population 1208 had multiple peaks as shown in FIG. 12C. Mapping the well locations to the fluorescence image of the chip as illustrated in FIG. 12E showed that they were localized. Referring to the fluorescence image, this location coincide with the location of bubbles. By excluding data from the wells near the bubbles, it made possible to identify sixteen wells that were erroneously detected as the G12A mutant and one well that was erroneously detected as the G13D mutant.

As described above, when identifying genotype in the digital PCR, erroneous identification can be prevented and the measurement accuracy can be improved, since the wells with erroneously calculated melting temperature may be excluded by detecting the bubbles based on the number of peaks of the melting curve and excluding data from the wells near the bubbles.

### EXPLANATION OF NUMBERS

- 101: DNA
- 102: Fluorescent-labeled probe
- 103: Fluorescent dye
- 104: Quencher
- 201: Droplet
- 202: Droplet capturing pit
- 203: Oil
- 204: Cartridge
- 205: Bubble
- 301: PCR solution
- 302: Through hole chip
- 303: Oil
- 304: Cartridge
- 305: Bubble
- 401: Tm
- 501: Tm of wild-type allele
- 502: Tm of mutant allele
- 503,: 504 Erroneously calculated Tm
- 601, 602, 611: Droplet
- 603: Micro-channel
- 604: Light source
- 605: Fluorescence filter
- 607: Imaging device
- 608: Lens
- 609: Dichroic mirror
- 610: Droplet detection cartridge
- 612: Temperature control stage
- 613: Cartridge
- 614, 615: Well
- 701: Fluorescence measurement unit
- 702: Computer
- 703: Analysis unit
- 704: Database
- 705: Memory
- 706: Monitor
- 1001: In-cartridge chip
- 1002: Estimated position of bubbles
- 1003: Tm range of wild-type allele
- 1004: Tm range of mutant allele
- 1005 curve: Plots of each microcompartment calculated by melting analysis
- 1201: Wells containing wild type-
- 1202: Wells containing G12A mutant -
- 1203: Wells containing G13D mutant -
- 1204: Tm range of wild-type allele
- 1205: Tm range of G12A mutant
- 1206: Tm range of G13D mutant
- 1207: Negative well
- 1208: Population with Tm outside the wild-type Tm range
- 1209 melting: Wells having multiple peaks in differential curve of curve

## Claims

1. A DNA detection method comprising:
a first step of dividing a specimen solution containing a fluorescent-labeled probe or a DNA intercalator and multiple types of DNA to be detected into a plurality of aliquots;
a second step of performing PCR in microcompartments, each containing one of the aliquots;
a third step of measuring fluorescence intensity from the fluorescent-labeled probe or the DNA intercalator in each of the microcompartments as temperature changes;
a fourth step of calculating a melting temperature of the multiple types of DNA to be detected from each measured fluorescence intensity;
a fifth step of identifying one or more of the microcompartments affected by one or more bubbles; and
a sixth step of excluding data obtained for the one or more of the microcompartments affected by one or more bubbles from all data obtained for the plurality of microcompartments.

2. The DNA detection method according to claim 1, wherein in the fifth step, the microcompartments, each having a number of peaks in a differential curve of a melting curve created from each fluorescence intensity measured in the third step, the number being greater than or equal to a predetermined number, are identified as the one or more of that microcompartments affected by one or more bubbles.

3. The DNA detection method according to claim 1 or 2, wherein in the fifth step, if two or more adjacent of the microcompartments are identified as the microcompartment affected by one or more bubbles, data of the two or more adjacent of the microcompartments are excluded in the sixth step; and
wherein in the fifth step, if a single of the microcompartments is identified as the microcompartments affected by one or more bubbles, data of the single one is not excluded in the sixth step.

4. The DNA detection method according to claim 1, wherein in the fifth step, the two or more adjacent of the microcompartments affected by one or more bubbles are identified by image analysis of the microcompartments.

5. The DNA detection method according to claim 1, wherein in the fifth step, the two or more adjacent of the microcompartments affected by one or more bubbles are identified by selecting the microcompartments, each having a number of peaks in a differential curve of the melting curves created from the fluorescence intensity measured in the third step, the number being greater than or equal to a predetermined number, followed by confirming whether the selected microcompartments are affected by one or more bubbles or not by image analysis of the microcompartments.

6. The DNA detection method according to claim 5, wherein in the fifth step, two or more adjacent of the microcompartments, each having a number of peaks in the differential curve, the number being greater than or equal to the predetermined number are selected ; and
wherein in the fifth step, a single one of the microcompartments having a number of peaks in the differential curve, the number being greater than or equal to the predetermined number is not selected.

7. The DNA detection method according to any one of claims 1 to 6, wherein in the first step, the solution containing the multiple types of DNA is subjected to limiting dilution.

8. The DNA detection method according to any one of claims 1 to 7, wherein in the fourth step, the type of DNA to be detected is identified in each of the microcompartments based on the melting temperature; and
wherein in the fifth step, the two or more of the microcompartments affected by one or more bubbles are identified, further based on a predetermined reference melting temperature for each of the identified type of DNA.

9. The DNA detection method according to any one of claims 1 to 8, wherein the microcompartments are constituted by wells arranged in an array or droplets dispersed in oil.

10. The DNA detection method according to any one of claims 1 to 9, wherein in the fourth step, the melting temperature is calculated as an inflection point in the melting curve created from each fluorescence intensity measured in the third step.

11. A DNA detection system comprising:
a first device having a microcompartment for comprising a DNA solution containing a fluorescent-labeled probe or a DNA intercalator;
a second device for capturing an image of the first device;
a third device for regulating temperature of the microcompartment in order to perform PCR in the microcompartment;
a fourth device for making the imaging device capture an image to acquire fluorescence intensity in the microcompartment as temperature changes; and
a fifth device for detecting generation of one or more bubbles from the acquired fluorescence intensity in the microcompartment.
